# EUROPEAN PATENT APPLICATION

(11) **EP 4 332 573 A2**
(43) Date of publication of application: **06.03.2024**
(21) Application number: 23193001.7
(22) Date of filing: 23.08.2023
(51) Int. Cl.: G01N 33/493

(54) **URINE ANALYSIS**

(30) Priority: 02.09.2022 US 202263403424 P; 17.08.2023 US 202318235318
(71) Applicant: Outsense Diagnostics Ltd., 6037604 Or Yehuda (IL)
(72) Inventor: KAPP-BARNEA, Yaara, 4480500 Nirit (IL)
(74) Representative: Barker Brettell LLP

(57) **Abstract**

Apparatus and methods are described including one or more sensors that are coupled to a toilet bowl, and that are configured to detect one or more urine-related parameters relating to a subject's urine, and at least one computer processor configured to receive the one or more urine-related parameters from the one or more sensors, determine when the subject's urine has an elevated creatinine concentration, and determine a likely cause of the elevated creatinine concentration. Other applications are also described.

## Description

This application claims priority benefit of U.S. Provisional Application No. 63/403,424 filed September 2, 2022, and U.S. Patent Application No. 18/25,318 filed August 17, 2023, which are hereby incorporated by reference in their entirety.

### FIELD OF EMBODIMENTS OF THE INVENTION

Some applications of the present invention generally relate to analysis of bodily emissions. Specifically, some applications of the present invention relate to apparatus and methods for detecting and classifying elevated creatinine in a subject's urine.

### BACKGROUND

Creatinine is a normal waste product of muscle that is ingested (e.g., by eating meat), as well as autologous muscle catabolism. Healthy kidneys remove creatinine from the blood, and creatinine leaves the body in urine. Creatinine is a relatively small molecule (60 Daltons) that distributes throughout total body water. The concentration of creatinine in urine may be indicative of muscle metabolism, diet, hydration state, and the capacity of the kidneys to perform glomerular filtration. The physiological creatinine concentration of a healthy person may vary according to one's body size and muscle mass, but normal creatinine concentration in urine is often within the range of 0.2 to 3.2 g/L.

### SUMMARY OF EMBODIMENTS

In accordance with some applications of the present invention, a sensor module is disposed inside a toilet bowl and includes one or more sensors that are configured to detect one or more urine-related parameters. For some applications, the one or more sensors detect at least some of the aforementioned parameters while the bodily emission is being emitted by the subject into the toilet bowl. Alternatively or additionally, the one or more sensors detect at least some of the aforementioned parameters when the bodily emission is disposed within the toilet bowl, subsequent to its emission by the subject. For some applications, the sensors include an imaging component, such as an RGB camera, a spectral camera, and/or a hyperspectral camera. Alternatively or additionally, the one or more sensors may include one or more light sensors that are configured to receive light from the bodily emissions. For some applications, the sensor module includes one or more illumination components. In accordance with respective applications, such illumination components include illumination components that are configured to illuminate the bodily emissions at given spectral bands (e.g., LEDs and/or lasers), and/or a broadband light source. For some applications, a broadband light source is used in combination with one or more bandpass filters (which may be used to filter the emitted and/or the detected light). For some applications, a computer processor receives the one or more urine-related parameters from the one or more sensors. For some applications, at least partially based upon the one or more urine-related parameters, the computer processor determines that the subject has elevated creatinine. For some applications, the computer processor determines the likely cause of the subject's elevated creatinine, by distinguishing between elevated creatinine (a) resulting from the subject's diet (e.g., from ingesting too much meat), (b) resulting from catabolic processes (i.e., a breakdown of the subject's muscles, which may be caused by a stress response to excessive exercise), (c) resulting from dehydration, and/or (d) resulting from kidney dysfunction (i.e., a problem with glomerular filtration).For some applications, the sensors are configured to detect light in the visible range and the computer processor is configured to determine that the subject has elevated creatinine based on the light that is detected in the visible range. For some applications, the toilet bowl is illuminated with broadband light and absorption of light within a given spectral range is detected in order to determine that the subject has elevated creatinine. For some such applications, the computer processor derives the concentration of creatinine by detecting an absorbance peak that is centered between a predetermined wavelength range such as 500 nm and 570 nm, between 510 nm and 550 nm, or in another range. For some applications, the computer processor derives the concentration of creatinine based upon the absorbance at the above-described absorbance peak, relative to absorbance at other wavelengths. For some applications, the computer processor identifies an optical signature of creatinine within the visible light range, e.g., within a spectral range of between 350 nm and 700 nm. For some such applications, the computer processor detects a ratio between (a) the light intensity at a wavelength band within this range at which creatinine is expected to have an absorbance peak (e.g., between 500 nm and 570 nm, and or between 510 nm and 550 nm), and (b) the light intensity at a wavelength band within this range at which creatinine is expected to have an absorbance minimum (e.g., between 600 and 680 nm, and/or between 360 nm and 440 nm). Alternatively or additionally, the one or more sensors are configured to detect light in the near-infrared and/or the mid-infrared range and the computer processor is configured to determine that the subject has elevated creatinine based on the light that is detected in the near-infrared and/or the mid-infrared range.

As described hereinabove, for some applications, pursuant to determining that the subject has elevated creatinine, the computer processor determines the likely cause of the subject's elevated creatinine, by distinguishing between elevated creatinine (a) resulting from the subject's diet (e.g., ingesting too much meat), (b) resulting from catabolic processes (i.e., a breakdown of the subject's muscles, which may be caused by caused by a stress response to excessive exercise), (c) resulting from dehydration, and/or (d) resulting from kidney dysfunction (i.e., a problem with glomerular filtration).

For some applications, based on the cause of elevated creatinine that was determined, the computer processor generates an output, e.g., indicating a recommended lifestyle change. For example, the computer processor may generate an output on a user interface device indicating a recommendation that the subject should consider making changes to their diet (in response to detecting elevated creatinine resulting from diet), that the subject should consider modifying their exercise regime (e.g., in response to detecting elevated creatinine resulting from catabolic processes), that the subject should drink more (e.g., in response to detecting elevated creatinine resulting from dehydration), and/or that the subject should seek medical attention (e.g., in response to detecting elevated creatinine resulting from dehydration or from kidney dysfunction).

For some applications, the sensor module and/or a user interface device communicates with a remote server. For example, the apparatus may communicate with a physician or an insurance company over a communication network without intervention from the subject. The physician or the insurance company may evaluate the output and determine whether further testing or intervention is appropriate for the subject. For some applications, data relating to the received sensor signals are stored in a memory. Periodically, the subject may submit the stored data to a facility, such as a healthcare facility (e.g., a physician's office, or a pharmacy) or an insurance company, and a computer processor at the facility may then perform the above-described analysis on a batch of data relating to a plurality of bodily emissions of the subject that were acquired over a period of time.

There is therefore provided, in accordance with some applications of the present invention, an apparatus including:
one or more sensors that are coupled to a toilet bowl, and that are configured to detect one or more urine-related parameters relating to a subject's urine, and
at least one computer processor configured to:
receive the one or more urine-related parameters from the one or more sensors,
determine when the subject's urine has an elevated creatinine concentration, and
determine a likely cause of the elevated creatinine concentration.

In some applications, the one or more sensors are configured to detect the one or more urine-related parameters relating to the subject's urine, without requiring any action to be performed by any person subsequent to emission of the urine into the toilet bowl.

In some applications, the one or more sensors include one or more light sensors that are configured to detect visible light, and the computer processor is configured to derive a concentration of creatinine in the subject's urine by detecting an absorbance peak that is centered between 500 nm and 570 nm, within the detected visible light.

In some applications, the one or more sensors include one or more light sensors that are configured to detect visible light, and the computer processor is configured to derive a concentration of creatinine in the subject's urine by:
determining light intensities of at least two spectral bands within the received light that are within a range of 350 nm and 700 nm, by analyzing the received light, and
determining a ratio of the intensities of the at least two spectral bands within the received light.

In some applications, the apparatus is configured for use with an output device, and, in response to determining the likely cause of the elevated creatinine concentration, the computer processor is configured to generate an output on the output device indicating a recommendation of a lifestyle change by the subject.

In some applications, the computer processor is configured to detect that the subject's urine has an elevated concentration of urinary NT-titin, and is configured to determine that the likely cause of the elevated creatinine concentration is catabolic processes at least partially in response to detecting that the subject's urine has the elevated concentration of urinary NT-titin.

In some applications, the apparatus is configured for use with an output device, and, in response to determining that the likely cause of the elevated creatinine concentration is catabolic processes, the computer processor is configured to generate an output on the output device indicating that the subject should modify their exercise regime.

In some applications, the one or more sensors include one or more light sensors that are configured to detect light in the range of between 190 nm and 250 nm, and the computer processor is configured to detect that the subject's urine has an elevated concentration of urinary NT-titin by detecting an optical signature of NT-titin within the range of between 190 nm and 250 nm.

In some applications, the computer processor is configured to detect a specific gravity of the subject's urine, and is configured to determine the likely cause of the elevated creatinine concentration at least partially in response thereto.

In some applications, at least partially in response to determining that the specific gravity of the subject's urine is low, the computer processor is configured to determine that the likely cause of the elevated creatinine concentration is kidney dysfunction.

In some applications, the apparatus is configured for use with an output device, and, in response to determining that the likely cause of the elevated creatinine concentration is kidney dysfunction, the computer processor is configured to generate an output on the output device indicating that the subject should seek medical attention.

In some applications, the computer processor is further configured to detect a concentration of urinary NT-titin of the subject's urine, and at least partially in response to determining that the concentration of urinary NT-titin and the specific gravity of the subject's urine are both within a normal range, the computer processor is configured to determine that the likely cause of the elevated creatinine concentration is dietary.

In some applications, the apparatus is configured for use with an output device, and, in response to determining that the likely cause of the elevated creatinine concentration is dietary, the computer processor is configured to generate an output on the output device indicating that the subject should modify their diet.

In some applications, at least partially in response to determining that the specific gravity of the subject's urine is elevated, the computer processor is configured to determine that the likely cause of the elevated creatinine concentration is either kidney dysfunction or dehydration.

In some applications, the computer processor is configured to determine a level of protein in the subject's urine, and is configured to determine the likely cause of the elevated creatinine concentration at least partially in response thereto.

In some applications, the computer processor is configured to derive a parameter that is indicative of foaming and/or turbidity of the subject's urine, and is configured to determine a level of protein in the subject's urine based on the derived parameter.

In some applications, at least partially in response to determining that the specific gravity of the subject's urine is elevated and that the level of protein in the subject's urine is elevated, the computer processor is configured to determine that the likely cause of the elevated creatinine concentration is kidney dysfunction.

In some applications, the apparatus is configured for use with an output device, and, in response to determining that the likely cause of the elevated creatinine concentration is kidney dysfunction, the computer processor is configured to generate an output on the output device indicating that the subject should seek medical attention.

In some applications, at least partially in response to determining that the specific gravity of the subject's urine is elevated and that the level of protein in the subject's urine is normal, the computer processor is configured to determine that the likely cause of the elevated creatinine concentration is dehydration.

In some applications, the apparatus is configured for use with an output device, and, in response to determining that the likely cause of the elevated creatinine concentration is dehydration, the computer processor is configured to generate an output on the output device indicating that the subject should drink more.

In some applications, the computer processor is configured to determine a level of protein in the subject's urine, and is configured to determine the likely cause of the elevated creatinine concentration at least partially in response thereto.

In some applications, the computer processor is configured to derive a parameter that is indicative of foaming and/or turbidity of the subject's urine, and is configured to determine a level of protein in the subject's urine based on the derived parameter.

In some applications, at least partially in response to determining that the level of protein in the subject's urine is elevated, the computer processor is configured to determine that the likely cause of the elevated creatinine concentration is kidney dysfunction.

In some applications, the apparatus is configured for use with an output device, and, in response to determining that the likely cause of the elevated creatinine concentration is kidney dysfunction, the computer processor is configured to generate an output on the output device indicating that the subject should seek medical attention.

There is further provided, in accordance with some applications of the present invention, a method including:
using one or more sensors, detecting one or more urine-related parameters relating to a subject's urine; and
using at least one computer processor:
receiving the one or more urine-related parameters from the one or more sensors,
determining when the subject's urine has an elevated creatinine concentration, and
determining a likely cause of the elevated creatinine concentration.

There is further provided, in accordance with some applications of the present invention, apparatus including:
one or more light sensors that are configured to receive light from a toilet bowl, while a subject's urine is disposed within the toilet bowl; and
a computer processor configured to:
determine light intensities of at least two spectral bands within the received light that are within a range of 350 nm and 700 nm, by analyzing the received light;
determine a ratio of the intensities of the at least two spectral bands within the received light;
in response thereto, determine that the subject has elevated creatinine within their urine.

The present invention will be more fully understood from the following detailed description of embodiments thereof, taken together with the drawings, in which:

### BRIEF DESCRIPTION OF THE DRAWINGS

FIG. 1 is a schematic illustration of apparatus for analyzing a bodily emission, in accordance with some applications of the present disclosure;

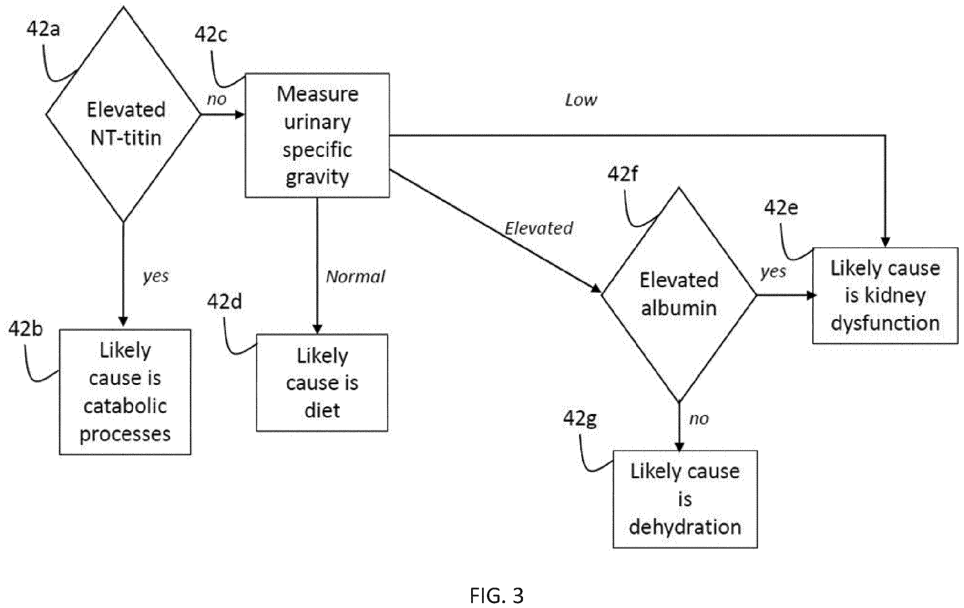

FIGS. 2 and 3 are flowcharts showing steps of analysis that is performed on a subject's urine, in accordance with some applications of the present disclosure;
FIG. 4 illustrates an example toilet including the apparatus for analyzing the bodily emission;
FIG. 5 illustrates another example toilet including the apparatus for analyzing the bodily emission;
FIG. 6 illustrates another example apparatus for analyzing the bodily emission;
FIG. 7 illustrates an example controller for the apparatus for analyzing the bodily emission; and
FIG. 8 illustrates an example flowchart for the examples in FIGS. 1-7.

### DETAILED DESCRIPTION OF EMBODIMENTS

As shown, for some applications, apparatus 20 includes a sensor module 22, which is placed inside a toilet bowl 23. For example, the apparatus 20 may be supported by or placed in proximity to the rim of the toilet bowl 23 For some applications, the sensor module 22 (and/or additional components of the apparatus) is integrated into the toilet bowl 23.For example, the sensor module 22 may be placed within the base of the toilet with a window in the toilet bowl 23 providing a view to the interior of the toilet bowl 23. The sensor module 22 includes one or more sensors 24. For some applications, the one or more sensors 24 are configured to detect one or more urine-related parameters (such as, voiding frequency, urine volume, urine color, and/or urine concentration, etc.), and/or to detect one or more feces-related parameters (such as, feces shape, size, texture, etc.). For some applications, the one or more sensors 24 detect at least some of the aforementioned parameters while the bodily emission is being emitted by the subject into the toilet bowl 23. Alternatively or additionally, the one or more sensors 24 detect at least some of the aforementioned parameters when the bodily emission is disposed within the toilet bowl 23, subsequent to its emission by the subject. For some applications, the sensors 24 include an imaging component, such as an RGB (red, green, blue) camera configured to detect light in the visible range, a spectral camera, and/or a hyperspectral camera. The spectral camera may be configured to detect light in multiple ranges of wavelengths or bands including the infrared, the visible spectrum, the ultraviolet, x-rays, or some combination of the above. The spectral camera may be configured to acquire image data in visible and non-visible bands simultaneously, illumination from outside the visible range, or the use of optical filters to capture a specific spectral range. The spectral camera may be configured to output data values for multiple wavelength bands for each pixel in an image. In a similar example, the hyperspectral camera may be configured to obtain the entire spectrum for each pixel in the image.

Alternatively or additionally, the one or more sensors may include one or more light sensors that are configured to receive light from the bodily emissions. For some applications, the sensor module 22 includes one or more illumination components 25. In accordance with respective applications, such illumination components include illumination components that are configured to illuminate the bodily emissions at given spectral bands (e.g., LEDs and/or lasers), and/or a broadband light source. For some applications, a broadband light source is used in combination with one or more bandpass filters (which may be used to filter the emitted and/or the detected light).

For some applications, a computer processor receives the one or more urine-related parameters from the one or more sensors, and/or receives the one or more feces-related parameters from the one or more sensors. In accordance with respective applications, the computer processor that performs the analysis described herein is a computer processor 28 disposed inside housing 30 (which may also house the sensor module 22), or is a different computer processor that is in communication with the sensor module 22.

For some applications, apparatus 20 includes a power source (e.g., a battery or other source of power), that is disposed outside the toilet bowl inside housing 30. Alternatively or additionally, the sensor module 22 is connected to mains electricity (e.g., utility source). For some applications, the power source and sensor module 22 are connected wiredly, or wirelessly. In accordance with respective applications, the computer processor that performs the analysis described herein is disposed inside the toilet bowl 23 (e.g., computer processor 28 disposed inside housing 30 (which may also house the sensor module 22)), or remotely. For example, as shown, the sensor module 22 may communicate wirelessly with a user interface device 32 that includes a computer processor. Such a user interface device may include, but is not limited to, a phone 34, a tablet computer 36, a laptop computer 38, or a different sort of personal computing device. For some applications the user interface device acts as both an input device and an output device, via which the user interacts with sensor module 22. The sensor module 22 may transmit data to the user interface device and the user interface device computer processor may run a program that is configured to analyze the received data.

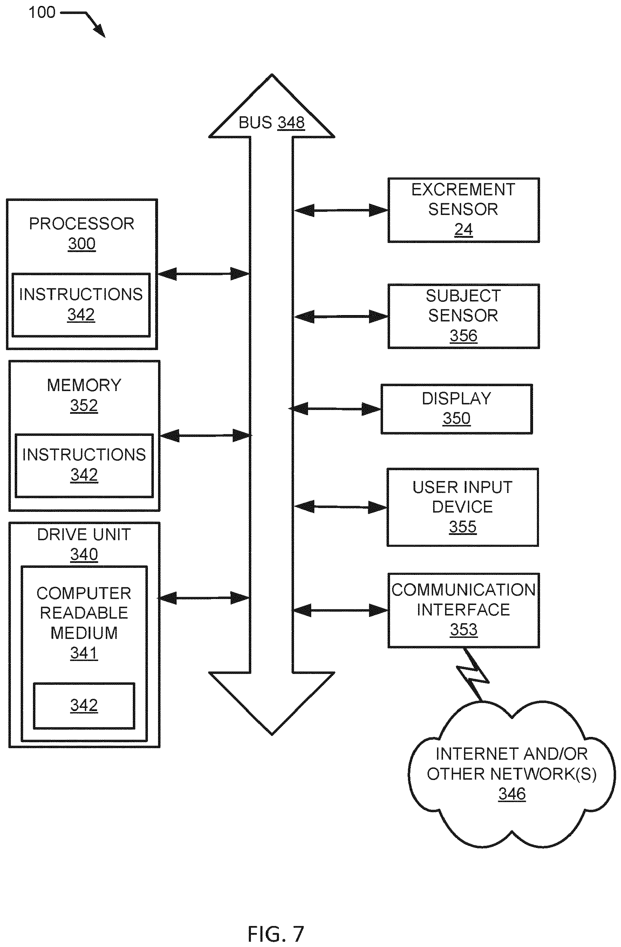

For some applications, sensor module 22 and/or the user interface device 32 communicates with a remote server (as shown by networks 346 in FIG. 7). For example, the apparatus may communicate with a physician or an insurance company over a communication network without intervention from the subject. The physician or the insurance company may evaluate the results and determine whether further testing or intervention is appropriate for the subject. For some applications, data relating to the received sensor signals are stored in a memory. For example, the memory may be disposed inside the toilet bowl 23 (e.g., inside the sensor unit), inside housing 30, or remotely. Periodically, the subject may submit the stored data to a facility, such as a healthcare facility (e.g., a physician's office, or a pharmacy) or an insurance company, and a computer processor at the facility may then perform the above-described analysis on a batch of data relating to a plurality of bodily emissions of the subject that were acquired over a period of time.

It is noted that the apparatus and methods described herein include a screening test in which the subject is not required to physically touch the bodily emission. Furthermore, for some applications, the subject is only required to touch any portion of the dedicated sensing apparatus periodically, for example, in order to install the device, or to change or recharge the device batteries. (It is noted that the subject may handle the user interface device, but this may be a device (such as a phone 34, a tablet computer 36, a laptop computer 38, or a different sort of personal computing device) that subject handles even when not using the sensing apparatus.) For some applications, the apparatus and methods described herein do not require adding anything to the toilet bowl 23 subsequent to the subject emitting a bodily emission into the toilet bowl 23, in order to facilitate the analysis of the emission, and/or a determination that the subject is suffering from dehydration and the classification thereof. For some applications, the subject is not required to perform any action after installation of the apparatus in the toilet bowl 23. The testing is automatic and handled by the apparatus, and monitoring of the subject's emissions is seamless to the subject and does not require compliance by the subject, so long as no abnormality is detected.

For some applications, subsequent to the subject emitting a bodily emission into the toilet bowl 23 (and optionally once the subject has finished excreting the bodily emission, and the bodily emission is at least partially disposed within the water of the toilet bowl 23), the bodily emission is imaged by receiving reflected and/or transmitted light from the toilet bowl 23, without requiring any action to be performed by any person subsequent to the emission. For some applications, the bodily emission is analyzed during the emission of the bodily emission into the toilet bowl 23. That is, sensor data may be collected while the bodily emission is falling between the subject and the toilet bowl 23.

For some applications, for each emission of the subject, in the case of any findings, the apparatus reports the findings to the subject via an output device, e.g., via user interface device 32. For some applications, the output device includes an output component (such as a light (e.g., a light-emitting diode (LED)) or a screen) that is built into apparatus 20.

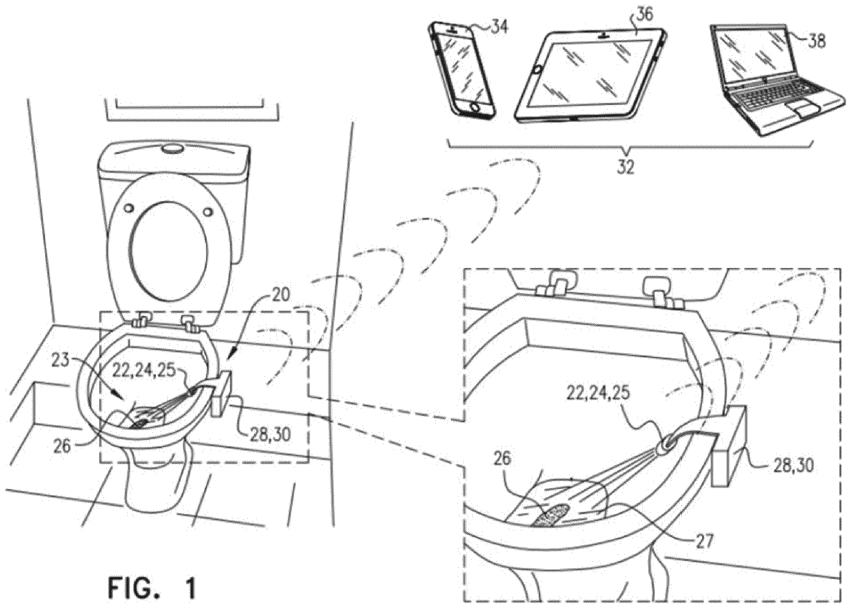

For some applications, sensor module 22 is disposed inside a toilet bowl 23. For some applications, the sensor module 22 includes an imaging component, which in turn includes one or more light sensors that are configured to receive light from bodily emissions that were emitted by the subject and are disposed inside the toilet bowl 23. For some applications, the sensor module 22 is housed in a water-resistant housing. For some applications, the face of the sensor module 22 underneath which the imaging component is mounted is covered with a transparent, water-resistant cover. It is noted that FIG. 1 shows the sensor module 22 disposed above the water level of the water within the toilet bowl 23. However, for some applications, at least a portion of the sensor module 22 (e.g., the entire sensor module 22) is submerged within the water in the toilet bowl 23.

For some applications, the sensor module 22 includes a subject sensor (e.g., as illustrated as sensor 356 in FIG. 7 or otherwise included in the sensor module 22). The subject sensor is configured to detect when a subject is on or in the vicinity of the toilet, and/or if the subject has defecated and/or urinated into the toilet bowl 23. For example, the subject sensor may include a motion sensor, configured to sense the motion of feces, urine, the subject, or the water in the toilet bowl 23. Alternatively or additionally, the subject sensor may include a light sensor configured to detect when the light in the bathroom is switched on, or when the subject sits on the toilet. For some applications, light sensors that are used for detecting light from the bodily emission are also used for the aforementioned function. For some such applications, the sensor module 22 is configured to be in standby mode most of the time (such that the sensor module 22 uses a reduced amount of power). The sensor module 22 is switched on in response to detecting that the subject is on or in the vicinity of the toilet, and/or that the subject has defecated and/or urinated into the toilet bowl 23. For some applications, the imaging and/or sensing components of the sensor module 22 acquire data in response to detecting that the subject is on or in the vicinity of the toilet, and/or that the subject has defecated and/or urinated into the toilet bowl 23. For some applications, the subject switches on the sensor module 22 manually.

For some applications, at least partially based upon the one or more urine-related parameters, the computer processor determines that the subject has elevated creatinine (e.g., creatinine in the sample above a predetermined creatinine level). For some applications, the computer processor determines the likely cause of the subject's elevated creatinine, by distinguishing between elevated creatinine (a) resulting from the subject's diet (e.g., from ingesting too much meat), (b) resulting from catabolic processes (i.e., a breakdown of the subject's muscles, which may be caused by caused by a stress response to excessive exercise), (c) resulting from dehydration, and/or (d) resulting from kidney dysfunction (i.e., a problem with glomerular filtration).

For some applications, the one or more sensors 24 are configured to detect light in the visible range and the computer processor is configured to determine that the subject has elevated creatinine based on the light that is detected in the visible range. For some applications, the toilet bowl 23 is illuminated with broadband light and absorption of light within a given spectral range is detected in order to determine that the subject has elevated creatinine. For some such applications, the computer processor derives the concentration of creatinine by detecting an absorbance peak that is centered between 500 nm and 570 nm (e.g., between 510 nm and 550 nm). For some applications, the computer processor derives the concentration of creatinine based upon the absorbance at the above-described absorbance peak, relative to absorbance at other wavelengths. For some applications, the computer processor identifies an optical signature of creatinine within the visible light range, e.g., within a spectral range of between 350 nm and 700 nm. For some such applications, the computer processor detects a ratio between (a) the light intensity at a wavelength band within this range at which creatinine is expected to have an absorbance peak (e.g., between 500 nm and 570 nm, and or between 510 nm and 550 nm), and (b) the light intensity at a wavelength band within this range at which creatinine is expected to have an absorbance minimum (e.g., between 600 and 680 nm, and/or between 360 nm and 440 nm).

Alternatively or additionally, the one or more sensors are configured to detect light in the near-infrared and/or the mid-infrared range and the computer processor is configured to determine that the subject has elevated creatinine based on the light that is detected in the near-infrared and/or the mid-infrared range. For example, the computer processor derives the concentration of creatinine by detecting light intensity at a spectral band that is centered between 2260 and 3000 nm, and/or light intensity at a spectral band that is centered between 1370 and 1410 nm.

For some applications, the computer processor determines that the subject's creatinine is elevated by detecting a concentration of creatinine in the urine that exceeds a threshold. For example, the threshold may be a concentration of between 3.3 and 4 g/L, e.g., 3.5 g/L, or 3.7 g/L. As described hereinabove, for some applications, pursuant to determining that the subject has elevated creatinine, the computer processor determines the likely cause of the subject's elevated creatinine, by distinguishing between elevated creatinine (a) resulting from the subject's diet (e.g., ingesting too much meat), (b) resulting from catabolic processes (i.e., a breakdown of the subject's muscles caused, which may be caused by caused by a stress response to excessive exercise), (c) resulting from dehydration, and/or (d) resulting from kidney dysfunction (i.e., a problem with glomerular filtration).

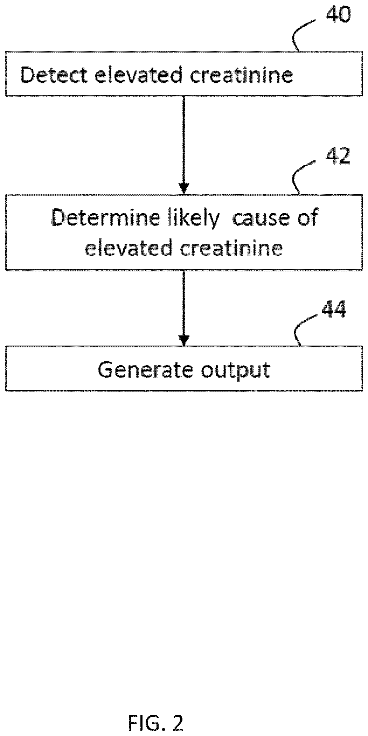

Reference is made to FIG. 2A, which is a flowchart showing steps of analysis that is performed on a subject's urine, in accordance with some applications of the present invention. For some applications, in a first step 40, the computer processor determines that the subject has elevated creatinine, for example, using the spectral analysis methods described hereinabove. Subsequently, in step 42, the computer processor determines the likely cause of the elevated creatinine, e.g., by distinguishing between elevated creatinine (a) resulting from the subject's diet (e.g., ingesting too much meat), (b) resulting from catabolic processes (i.e., a breakdown of the subject's muscles, which may be caused by caused by a stress response to excessive exercise), (c) resulting from dehydration, and/or (d) resulting from kidney dysfunction (i.e., a problem with glomerular filtration). For some applications, based on the cause of elevated creatinine that was determined in step 42, the computer processor generates an output, in step 44. For some applications, the computer processor generates an output indicating a recommended lifestyle modification. For example, the computer processor may generate an output on the user interface device indicating that the subject should consider making changes to their diet (in response to detecting elevated creatinine resulting from diet), that the subject should consider modifying their exercise regime (e.g., in response to detecting elevated creatinine, which may be caused by a stress response to excessive exercise ), that the subject should drink more (e.g., in response to detecting elevated creatinine resulting from dehydration), and/or that the subject should seek medical attention (e.g., in response to detecting elevated creatinine resulting from dehydration or from kidney dysfunction).

As described hereinabove, for some applications, sensor module 22 and/or the user interface device communicates with a remote server. For example, the apparatus may communicate with a physician or an insurance company over a communication network without intervention from the subject. The physician or the insurance company may evaluate the output and determine whether further testing or intervention is appropriate for the subject. For some applications, data relating to the received sensor signals are stored in a memory. For example, the memory may be disposed inside the toilet bowel (e.g., inside the sensor unit), inside housing 30, or remotely. Periodically, the subject may submit the stored data to a facility, such as a healthcare facility (e.g., a physician's office, or a pharmacy) or an insurance company, and a computer processor at the facility may then perform the above-described analysis on a batch of data relating to a plurality of bodily emissions of the subject that were acquired over a period of time.

Reference is now made to Fig. 2B, which is a flowchart shows a sequence of tests that are performed in order to determine the likely cause of elevated creatinine (i.e., step 42 of Fig. 2A), in accordance with some applications of the present invention. It is noted that the sequence in which these steps are performed do not necessarily follow the order in which they are shown in Fig. 2B. In addition, in some cases, in order to determine the likely cause of elevated creatinine, only a portion of the steps shown in Fig. 2B are performed. The scope of the present disclosure includes performing one or more of the steps described with reference to Fig. 2B in any sequence, in order to determine the likely cause of elevated creatinine.

One of the possible causes of elevated creatinine is catabolic processes (i.e., the breakdown of a person's muscles, which may be caused by a stress response to excessive exercise). Often, such catabolic processes also leads to the breakdown of Titin to urinary titin N-fragments (also known as NT-titin). Therefore, if the cause of the subject's elevated creatinine is excessive exercising, the subject would also be expected to exhibit elevated urinary NT-titin. For some applications, in step 42a, the computer processor determines whether the subject's urine has elevated concentration of urinary NT-titin. For some such applications, the one or more sensors 24 are configured to detect light in the range of between 190 nm and 250 nm (e.g., between 200 nm and 240 nm). Within this range, NT-titin has an optical signature comprising a peak at approximately 200 nm, a trough between 210 and 230 nm, and another peak at approximately 240 nm. For some applications, the computer processor determines derives the concentration urinary NT-titin based upon the absorbance that exhibits that above-described optical signature, relative to absorbance at other wavelengths. For some applications, in response to determining that the subject has elevated urinary NT-titin (e.g., by detecting that the concentration of urinary NT-titin exceeds a predetermined threshold, such as a threshold of between 1.5 and 20 ng/ml), the computer processor determines that the subject's elevated creatinine is at least partially caused by catabolic processes (step 42b).

Urinary specific gravity measures the concentration of solutes in the urine, by measuring the ratio of urine density compared with water density. For some applications, in step 42c, the computer processor measures the subject's urinary specific gravity. Healthy adult urine will often have a specific gravity in the range of 1.010 to 1.030. For some applications, the sensor module 22 is configured to generate a signal that is indicative of the absorption of light by the subject's urine, and the computer processor derives the specific gravity of the subject's urine based upon the signal. For some applications, the computer processor derives the specific gravity of the subject's urine based upon a sensor signal that is indicative of the absorption of cyan-green light (e.g., light within a wavelength band of 480-520 nm), by the subject's urine.

Two possible causes of elevated creatinine are dehydration and/or kidney dysfunction. For some applications, both of these conditions give rise to abnormal urinary specific gravity of the subject's urine, in addition to elevated creatinine concentration. Kidney dysfunction can give rise to either elevated or reduced urinary specific gravity, whereas dehydration often gives rise to elevated urinary specific gravity. Dietary factors (e.g., ingesting too much meat) do not typically lead to elevated urinary specific gravity. Therefore, if the computer processor determines that the subject's urinary specific gravity is within a predetermined range (e.g., within the range of 1.010 to 1.030) and the subject does not have elevated urinary NT-titin, for some applications, the computer processor determines that the likely cause of the elevated creatinine is diet (step 42d). If the computer processor determines that the subject's urinary specific gravity is reduced relative to a predetermined normal range (e.g., it is below a predetermined threshold, such as a threshold that is 1.010 or less, e.g., 1.010, or 1.005), for some applications, the computer processor determines that the likely cause of the elevated creatinine is kidney dysfunction (step 42e).

If the computer processor determines that the subject's urinary specific gravity is elevated relative to a predetermined normal range (e.g., it is above a predetermined threshold, such as a threshold that is 1.030 or more, e.g., 1.030, 1.035, or 1.040), then the cause of the elevated creatinine could be either kidney dysfunction or dehydration. For some applications, in order to distinguish between elevated creatinine that is caused by dehydration and elevated creatinine caused by kidney dysfunction, the computer processor determines whether the subject has elevated levels of protein (e.g., albumin) in their urine (step 42f). Protein (e.g., albumin) is normally abundant in the blood. Often, kidney dysfunction would give rise to elevated urinary protein (e.g., albumin), whereas dehydration would not.

If there is a problem with kidneys, protein can leak into urine, such that a large amount of protein in urine often indicates kidney disease. Although protein in urine tends not to have an optical signature, it has been observed that the presence of protein within a subject's urine can give rise to foaming of the urine because the protein has a soap-like effect that decreases the surface tension of urine. Expulsion of urine causes interaction of the electrostatic forces between molecules within the liquid and surface, leading to the formation of bubbles as a result of dispersion of air in the urine. For some applications, the computer processor is configured to detect a sensor signal that is indicative of foaming and/or turbidity of the subject's urine, and/or an amount of foaming and/or turbidity of the urine, and is configured to determine that the subject has an elevated concentration of protein in her/his urine at least partially in response thereto. Alternatively or additionally, the computer processor is configured to estimate a concentration of protein in the subject's urine, in response to foaming and/or turbidity of the subject's urine, and/or an amount of foaming and/or turbidity of the urine. For some such applications, the computer processor is configured to estimate turbidity of the urine by detecting opacity of the urine. For some applications, the computer processor is configured to measure a height of a foam layer above the urine, and to determine that the subject has an elevated concentration of protein in her/his urine, and/or estimate a concentration of protein in the subject's urine, in response thereto.

For some applications, in response to detecting an indication that the subject has an elevated concentration of protein in their urine (e.g., by deriving a concentration of protein that exceeds a threshold, by detecting a level of opacity that exceeds a threshold, a level of foaming that exceeds a threshold, and/or a level of turbidity that exceeds a threshold), the computer processor determines that the likely cause of the elevated creatinine is kidney dysfunction (step 42e). For some applications, in response to detecting an indication that the subject does not have an elevated concentration of protein in their urine, in combination with the subject's urinary specific gravity being elevated, the computer processor determines that the cause of the elevated creatinine is dehydration (step 42g).

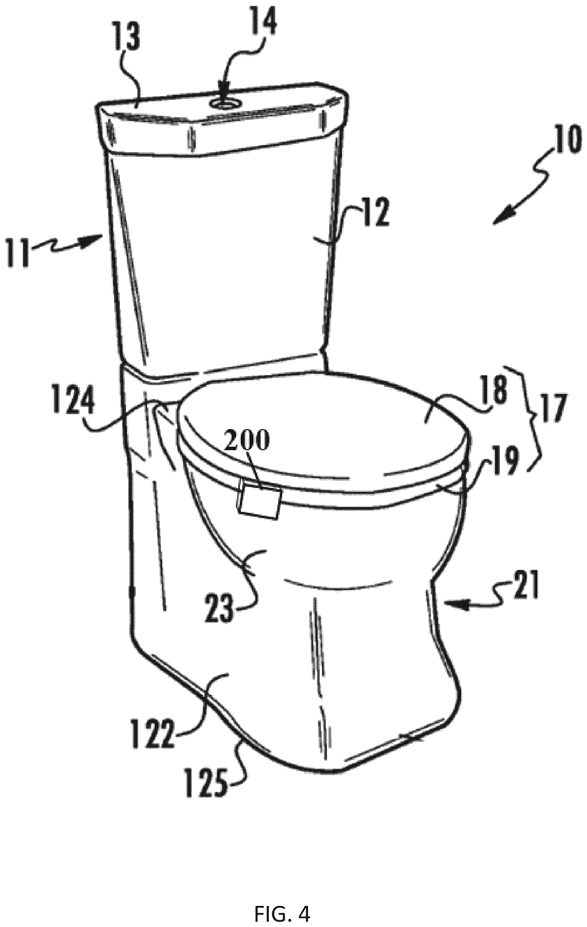

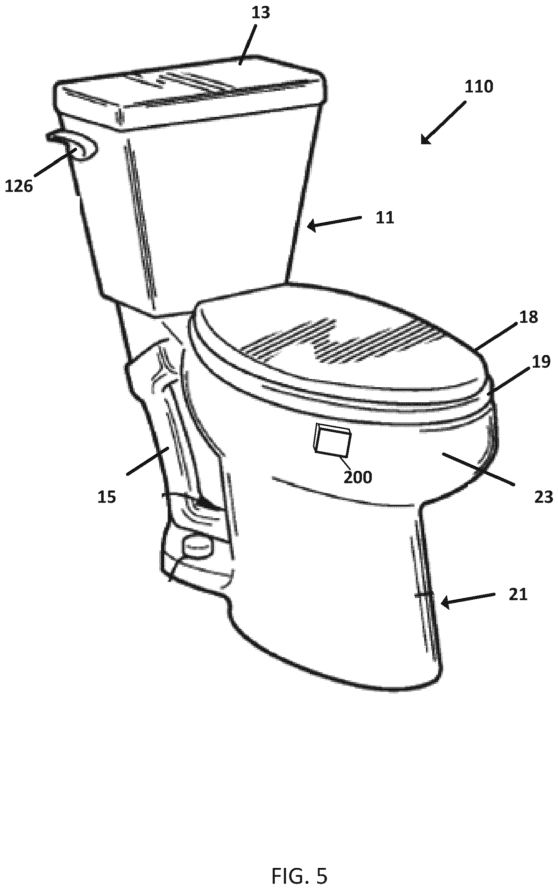

FIGS. 4 and 5 illustrate example toilets 10 including the apparatus for analyzing the bodily emission. The toilet 10 may include a biometric toilet data collection device 200, which may be configured using the structure and functions described herein for apparatus 20 and sensor module 22. The biometric toilet data collection device 200 may be removable. The biometric toilet data collection device 200 may be incorporated or physically coupled into either of the example toilets 10 of FIGS. 4 and 5. For example, mounting hole through the toilet 10 at the bowl may separate the sensor portion of the biometric toilet data collection device 200 from the processing and/or battery portion of the biometric toilet data collection device 200. A mounting rod coupled to the sensor portion may pass through the mounting hole and screw into or otherwise couple with the processing and/or battery portion of the biometric toilet data collection device 200. The biometric toilet data collection device 200 may also be mounted magnetically. The sensor portion of the biometric toilet data collection device 200 may include one magnet which attracts a second magnet including in the processing and/or battery portion of the biometric toilet data collection device 200. Data may be exchanged between the two portions inductively or through another wireless technique. In other examples, the biometric toilet data collection device 200 may be incorporated into the toilet seat assembly. FIG. 5 illustrates such as a mounting of the biometric toilet data collection device 200 below the rim of the toilet.

In other examples, the biometric toilet data collection device 200 may be mounted in a cavity in the biometric toilet data collection device 200 created from an insert mole and covered with a transparent or semi-transparent window.

FIG. 4 illustrates an exemplary embodiment of a skirted toilet 10 that includes a tank 11, a pedestal 21 (or base), a seat assembly 17 and a coupling or mounting assembly. The tank 11 may include a hollow container 12 for storing the water used during operational (or flushing) cycles, a lid (or cover) 13 for providing selective access into the container 12, and an actuator 14 that is configured to initiate an operational cycle when activated. The actuator 14 or flush mechanism may be a button configured to activate when depressed (or pulled) a predetermined distance or when touched, a lever configured to activate when rotated a predetermined angular travel, or any suitable device configured to activate based upon an input manipulation by a user. The actuator 14 may include multiple buttons, levers, or inputs, such that multiple flush types may be selected. The flush types may include a low volume flush and a high volume flush. A medium volume flush may also be included.

It should be noted that the shapes and configurations of the tank, pedestal, seat assembly, and the internal components (including the trapway and other features) may vary from the embodiments shown and described herein, and that the embodiments disclosed herein are not intended as limitations. Various components of the toilet may be made of vitreous china. Various components of the toilet may be polymeric and/or over molded or otherwise fixed to the toilet. It should be noted, for example, that although the exemplary embodiment of the toilet 10 is shown configured with the tank 11 formed separately from the pedestal 21 and later coupled to the pedestal, the tank may be integrally formed with the pedestal as a one-piece design. In other words, the toilet may be a one-piece design, a two-piece design, or have any suitable configuration. The toilet disclosed herein may have a wide variety of skirted toilet configurations, and all such configurations are intended to be encompassed herein. The following description of various toilet features is therefore intended as illustration only of one possible embodiment, and it should be understood by those reviewing the present description that similar concepts or features may be included in various other embodiments.

The tank 11 may include an inlet opening configured to receive water from a coupled water supply, such as from a hose (e.g., line, tube). The tank 11 may also include an inlet valve assembly or other device configured to control the flow of water from the water supply into the tank 11 through the inlet opening. Within the tank 11 may be provided a float device for controlling the inlet valve assembly, such as by opening the valve to refill the tank 11 of the tank 11 after an operational cycle and closing the valve when the water in the tank 11 reaches a preset volume or height. The tank 11 may also include an outlet opening configured to transfer (e.g., conduct) the water stored in the tank 11 of the tank to the pedestal 21 upon activation of the actuator 14. The pedestal 21 may include toilet bowl 23. The tank 11 may include an outlet valve assembly or other device configured to control the flow of water from the tank into the pedestal 21 through the outlet opening.

The pedestal 21 (or base) of the toilet 10 may include a wall 122 having any suitable shape that is configured to form the toilet bowl 23 having an opening formed by an upper rim at the top of the opening. The pedestal 21 may also be configured to include a plurality of walls having varying shapes that together form a bowl having an opening formed by a rim. The wall 122 of the pedestal may extend downward and/or rearward from the bowl 23 to form a lower portion 125 configured to support the pedestal 21 and the toilet 10. The lower portion 125 may be formed by the end (e.g., lower rim) of the wall 122, or may include a member that extends generally in a horizontal plane from one or more than one end of the wall. The pedestal 21 may also include a top member 124 that extends between two sides of the wall 122 (or between two opposing walls) and is provided rearward (or behind) the bowl 23, wherein the top member 124 forms a plateau for supporting the tank 11, such as the bottom surface of the tank 11. The top member 124 may include an inlet opening that may be aligned with the outlet opening of the tank 11, such as when the tank 11 is coupled to (or resting above) the pedestal 21, wherein water is selectively transferred (e.g., conducted) from the tank 11 through the outlet opening of the tank to the pedestal 21 through the inlet opening of the pedestal 21, when the toilet is activated through the actuator 14. The outlet valve assembly may control the flow of water from the tank to the pedestal. The toilet may also include a gasket or seal that is provided between the tank 11 and the pedestal 21 to prohibit leaking. For example, a gasket may be provided between the outlet opening of the tank and the inlet opening of the pedestal to prohibit leaking between the tank and the pedestal.

The plateau formed by the top member 124 of the pedestal 21 may also provide for coupling of the seat assembly 17 to the pedestal 21 of the toilet 10. For example, the top member 124 may include one or more than one opening, wherein each opening is configured to receive a fastening device (e.g., bolt, screw, etc.) to couple (e.g., attach) the seat assembly 17 to the top member 124 of the pedestal 21. As another example, the top member 124 may include one or more than one fastening device (e.g., bolts, recessed nuts, etc.) integrally formed therein (i.e., already provided connected or coupled to the pedestal 21), wherein the fastening device may be used to couple or secure at least a portion of the seat assembly 17 to the pedestal 21.

The top member 124 of the pedestal 21 may also be shaped to support the biometric toilet data collection device 200, which may be configured using the structure and functions described herein for apparatus 20 and sensor module 22. The toilet data collection device 200 may include at least one supporting element configured to be supported by the top member 124. The top member 124 may include one or more than one opening, wherein each opening is configured to receive a fastening device (e.g., bolt, screw, etc.) to couple (e.g., attach) the toilet data collection device 200 to the top member 124 of the pedestal 21. As another example, the top member 124 may include one or more than one fastening device (e.g., bolts, recessed nuts, etc.) integrally formed therein (i.e., already provided connected or coupled to the pedestal 21), wherein the fastening device may be used to couple or secure at least a portion of the top member 124 to the pedestal 21. The toilet data collection device 200 may rest on the surface of the top member 124 and be held in place through friction or a spring force.

The bowl 23 of the pedestal 21 may be configured to include a receptacle (e.g., sump) and an outlet opening, wherein the water and waste is collected in the receptacle until being removed through the outlet opening, such as upon activation of the actuator 14. The pedestal 21 may also include a pedestal internal passageway, such as a trapway, that connects the outlet opening or discharge outlet of the bowl 23 to a drain or soil pipe. The passageway, or trapway, generally includes a first portion, a second portion, and a weir separating the first and second portions. The first portion of the passageway may extend from the outlet opening of the bowl 23 at an upwardly oblique angle to the weir. The second portion of the passageway may extend from the weir downwardly to the exiting device, such as the drain or soil pipe.

Between operational cycles (e.g., flush cycles) of the toilet 10, the water (and waste) is collected in the first portion of the trapway (in addition to the receptacle of the bowl), such that the weir prohibits the water from passing past the weir and into the second portion of the trapway. A flushing cycle may begin upon activation of the actuator 14. Upon activation of the actuator, additional water (e.g., fresh water and or grey water) may be discharged into the bowl 23 of the pedestal 21, resulting in the flushing action and waste removal through the soil pipe. The flushing cycle may include generation of a siphon to assist the flushing action and waste removal.

The seat assembly 17 may include a cover member 18 (e.g., lid), a seat member 19 (e.g., ring member), and a hinge. The seat member 19 may be configured to include an annular member that encircles an opening, wherein the annular member provides a seating surface for the user of the toilet 10. The seat member 19 may also be pivotally coupled (e.g., attached) to the hinge, wherein the seat member may rotate (or pivot) about the hinge, such as between a first lowered or seated position and a second raised or upright position. The cover member 18 may be round, oval, or any other suitable shape. Typically, the profile or shape of the outer surface of the cover member will be configured to match (i.e., to be substantially similar) to the profile of the outer surface of the seat member to improve the aesthetics of the seat assembly and toilet. The cover member 18 may also be coupled to the hinge, wherein the cover member may rotate (or pivot) about the hinge, such as between a first down lowered or down position and a second raised or upright position. The cover member 18 may be provided above the seat member 19 in the down position to thereby cover the opening of the seat member 19, as well as to conceal the inside of the bowl 23 of the pedestal 21. The cover member 18 may be configured to rest against the outside surface of the tank 11, when the cover member 18 is in the upright position, such that the cover member 18 remains in the upright position in order for a user to sit upon the seat member 19. In one alternative, the seat assembly 17 may include the toilet data collection device 200 mounted behind a window.

As described below, the seat member 19 and/or the cover member 18 may be coupled to a sensor and/or a drive mechanism for initiating a flush cycle or a particular portion of the flush cycle, which may involve release of water from the tank 11 or actuation of a component of the flushing assembly.

FIG. 5 illustrates a non-skirted toilet 110 according to another exemplary embodiment of the present disclosure. Throughout this disclosure "toilet 10" may be used to refer to the toilet 10 of Figure 1 or the toilet 110 of Figure 20 in the alternative. In other words, any of the following embodiments may be applied to both toilet 10 and toilet 110. The internal components, including the trapway 15, are visible in the pedestal 21 of non-skirted toilet 20. It should be noted that the devices, methods, and systems described herein may include and/or be used with both skirted and non-skirted toilets.

FIG. 5 also includes flush lever 126, which is configured to receive input from a user for activation of a flush cycle. The flush lever 126 may be physically connected to an outlet valve, such as a flapper, for discharging water from the tank 11 into the toilet bowl 23. The flush lever 126 also may be electrically connected to a controller such that a sensor associated with (e.g., within) the flush lever 126 may generate data indicative of detection of the user or movement of the flush lever 126. The controller, in response, may generate a command to open the outlet valve.

The actuator 14 or the flush lever 126 may also be connected or in communication with any of the flush assemblies described herein. That is, in addition to operation of the outlet valve, user input to the actuator 14 or the flush lever 126 may cause one or more operations to occur in the following flush assemblies. In some examples, the user input to the actuator 14 may first operate the flush assembly and then cause operation of the outlet valve. In other examples, the flush assembly and the outlet valve are actuated simultaneously or substantially simultaneously. Substantially simultaneously may mean within a time period such that the time period is relatively small relative to the time of the flush cycle. Example time periods may include 1 seconds, 100 milliseconds, or 10 milliseconds. The operation of the flush assembly may cause water collected in the first portion of the trapway (in addition to the receptacle of the bowl), to pass past a weir and into the second portion of the trapway.

The flush lever 126 or actuator 14 may include or be replaced with a variety of other sensor types configured to produce an output signal indicative of a user input for a flush cycle. A magnetic sensor may be incorporated into the flush lever 126 or the actuator 14 to generate an electronic signal (e.g., sensor data) in response to actuation. The magnetic sensor may be imbedded into the flush lever 126 or the actuator 14 (e.g., hall-effect sensor) to detect movement. An optical sensor may detect nearby movement of a user (e.g., hand gesture, presence of a hand or user). The flush lever 126 or the actuator 14 may be tied to a reed sensor or other mechanical sensor that detects movement.

The flush lever 126 or the actuator 14 may include a button or multiple buttons that the user may depress to generate the output signal. In addition or in the alternative to the flush lever 126 or the actuator 14, a touch screen may receive user input and generate the output signal. In some examples, the touch screen may be capacitive. Other capacitive sensors may be incorporated directly onto the toilet 10.

The flush lever 126 or actuator 14 may include communication with another input device such as a remote control. The remote control may be a standalone device that is battery operated and communicates with a controller or with the flush lever 26 or the actuator 14. The external input device may be a smartphone, tablet, computer, or other mobile device.

In any of these examples, the flush lever 126, actuator 14, or standalone device may communicate with the toilet data collection device 200 in order to reset or otherwise affect the collection of data at the toilet data collection device 200. For example, when the flush cycles is started by the flush lever 126, actuator 14, or standalone device, the toilet data collection device 200 may stop any current collection of data for bodily emission and return to a standby state. In the standby state, the toilet data collection device 200 may wait for the subject sensor to detect when a subject (user) is on or in the vicinity of the toilet 10, and/or if the subject has defecated and/or urinated into the toilet bowl 23. That is, the toilet data collection device 200 may be placed in standby state by the flush lever 126 and then exit the standby state to a data collection mode based on a motion sensor, configured to sense the motion of feces, urine, the subject, or the water in the toilet bowl 23. Alternatively or additionally, the toilet data collection device 200 may be return to data collection mode from the standby state based on a light sensor configured to detect when the light in the bathroom is switched on, or when the subject sits on the toilet.

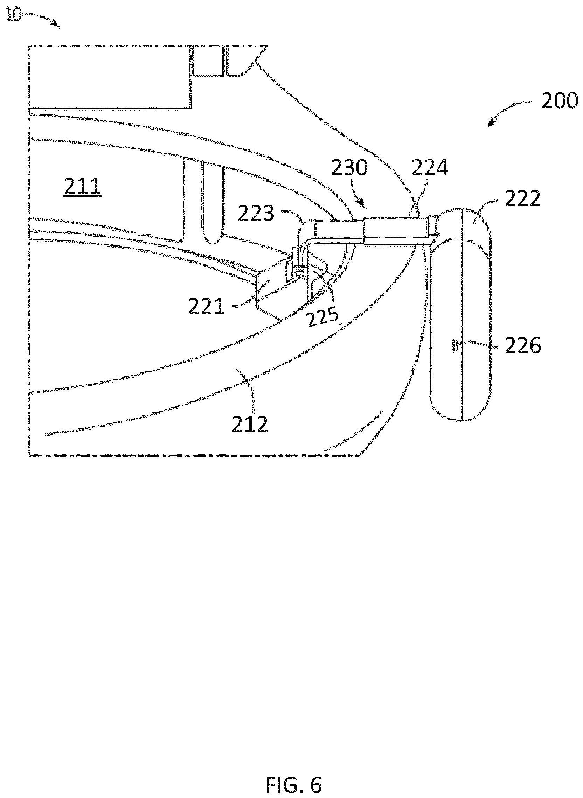

FIG. 6 illustrates a more detailed view of an example toilet data collection device 200 for analyzing the bodily emission. The toilet data collection device 200 may include one or more mechanisms for securing the toilet data collection device 200 to the rim of the toilet 10 with minimal moving parts. Toilets, and specifically toilet rims, are available in a variety of sizes and widths. In addition, the width of the toilet rim may vary around the circumference of the toilet. Thus, the data collection device may be adjustable to fit the various sizes of toilet rims. In the first embodiment, a spring loaded sleeve allows the collection device to adjust to various rim sizes. In a second embodiment, a modular extender may be interchangeable to accommodate different rim sizes. In a third embodiment, the collection device may be flexible and confirm to the size of the rim.

FIG. 6 illustrates toilet data collection device 200 in an unmounted position with respect to a toilet 10. The data collection device 200 may include a first compartment 221 and a second compartment 222. The first compartment 221 is coupled to the second compartment 222 by a bridge 230. The second compartment 222 may include a data port 226 to interface with another device. Additional, different, or fewer components may be included.

The bridge 230 includes an outer extender 224 and an inner extender 223 as sleeves such that the inner extender 223 fits within the outer extender 224. The inner extender 223 may be coupled to the first compartment 221. The outer extender 224 may be coupled to the second compartment 222. A spring inside the outer extender 224 may be configured to push the inner extender 223 to slide farther within the outer extender 224. The spring may pull the inner extender 223 with respect to the outer extender 224. The spring may pull the inner extender 223 toward the rim 12. The spring may be a constant force spring should that the force placed on the inner extender 223 and/or the outer extender 224 is constant. Other examples may include progressive springs where the force applied by the spring is proportional to the relative distance between the inner extender 223 and the outer extender 224. The progressive spring or springs includes a spring constant that is variable depending on the relative position of the inner extender 223 and outer extender 224. In one alternative, the inner extender 223 and/or the outer extender 224 includes a ratcheting device that allows the inner extender 223 and the outer extender 224 to be pressed together but not a part. The ratcheting mechanism may be released via a release button.

In the second embodiment, the bridge is modular or replacement. The bridge may be interchangeable such that a bridge of a first sized may be removed and replaced with a bridge of a second size. The bridge may be removable from the first compartment 221 and that of the second compartment 222. The bridge may snap-fit into a recess in each of the first compartment 221 and that of the second compartment 222. The bridge may be fastened to the first compartment 221 and/or the second compartment 222 using a screw, a pin, a spring, or another fastener. The bridge may be magnetically coupled to the first compartment 221 and/or the second compartment 222.

The user may interchange the bridge to select the bridge that fits a specific rim. A particular size for the bridge 31 may be selected based on the width of the rim. For example, a user may measure the width of the rim and place an order for the data collection device 20 having a bridge of the corresponding width. In one example, the user may place an order over the phone or website according to a particular model of toilet, and the size of the bridge is looked up according to the model of toilet.

The inner extender 223 and/or the first compartment 221 may include a bumper or grip 225 configured to press against the rim and hold the data collection device 200 to the toilet 10. The user may manually press the inner extender 223 and the outer extender 224 toward one another to cause the grip 225 to contact the rim 12 and hold the data collection device 200 to the toilet 10.

The first compartment 221 includes at least one circuit component and the second compartment 222 includes at least one circuit component. The bridge 230 includes wires, traces, or other conductors for provided power and/or communication between the circuitry of the first compartment 221 and that of the second compartment 222.

Because the bridge 230 changes size, by virtue of the relative movement of the inner extender 223 and the outer extender 224, the conductors should be adaptable. In some examples, the conductors may be provided by a ribbon cable that is configured to elongate or contract. In some examples, the conductors may be provided by looped or slacked wiring in the bridge such that the wires move within a cavity as the bridge 230 changes size. In some examples, the conductors may include traces on the inner extender 223 and the outer extender 224 that slide with respect to each other in a direction parallel so as to maintain the physical conduct and electrical conduct and the inner extender 223 is moved relative to the outer extender 224.

In other words, as the bridge 230 is adjusted in size, one or more electrical conductors (e.g., power connection and/or data connection) are also modified in respond to the adjustment in size of the bridge 230. In some examples, the one or more electrical conductors include a first electrical connection corresponding to a first size (e.g., reduced size) of the bridge 230 and a second electrical connection corresponding to a second size (e.g., extended size) of the bridge 230.

FIG. 7 illustrates an example control system 100 for any of the embodiments described herein for analyzing bodily emissions. The controller 100 may include a processor 300, a memory 352, and a communication interface 353 for interfacing with devices or to the internet and/or other networks 346. In addition to the communication interface 353, a sensor interface may be configured to receive data from the sensors described herein or data from any source. The controller 100 may include an integrated display 350, or other output devices. The components of the control system may communicate using bus 348. The control system may be connected to a workstation or another external device (e.g., control panel) and/or a database for receiving user inputs, system characteristics, and any of the values described herein.

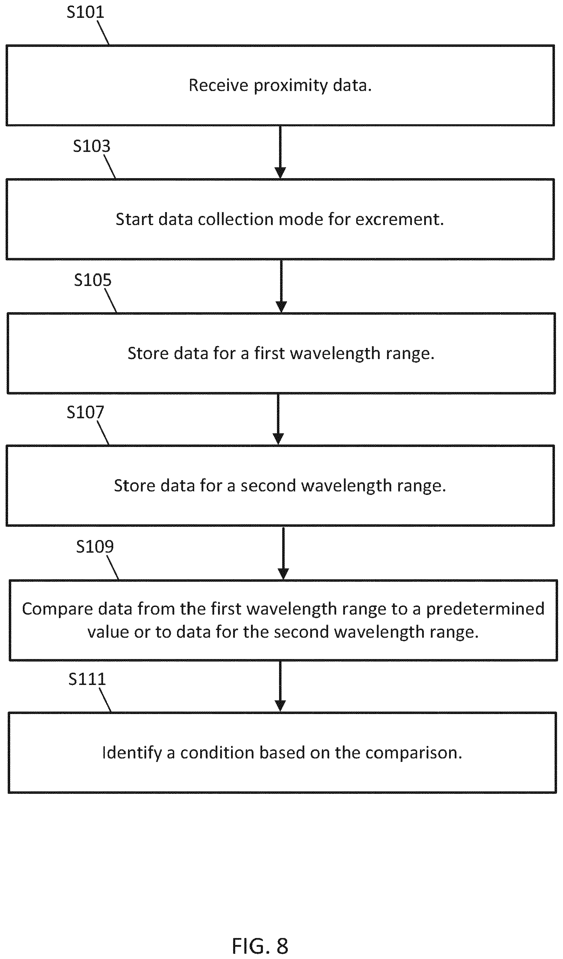

Figure 8 illustrates an example flow chart for the operation of the biometric data collection device using control system 100. Additional, different, or fewer acts may be performed.

At act S101, the toilet data collection device 200 is in a standby state where excrement data is not being collected, and proximity data is received from the subject sensor 356. The data from the subject sensor 356 indicates that a user has approached the toilet, sat on the toilet, or is otherwise in proximity to the toilet. The subject sensor 356 may detect presence, motion, temperature, or another characteristic of the user. The subject sensor 356 may detect a face of the user. The subject sensor 356 may detect identifying info for the user.

The subject sensor 356 may communicate with a personal device (e.g., phone) of the user to receive the identifying information for the user using a wireless communication protocol. Examples of wireless communication include the family of protocols known as Bluetooth. The Bluetooth protocol, or another ad hoc network, may allow for the direct connection probe without user intervention. For example, a Bluetooth transceiver, which is another example of a network device, may transmit or broadcast a packet of information that advertises the existence of the Bluetooth transceiver as available for an ad hoc connection. The existence of the direction connection probe, or put another way, the ability of the radio of the network device to receive the direction connection probe from the mobile device, indicates that the mobile device is in proximity. Other examples of wireless communication include radio frequency identification (RFID) or near field communication (NFC). These short range communication signals may be received when a mobile device passes near the receiver.

At act S103, the control system 100 begins a data collection mode in response to the data received by the subject sensor 356. In the data collection mode, the excrement sensor 24 generates data for the urine and/or feces that may be in the bowl 23 or in the vicinity of the toilet data collection device 200 (e.g., in air). The excrement sensor 24 may generate data based on light in one or more wavelength ranges. The excrement sensor 24 may detect light across multiple wavelength ranges. Multiple sensors may be used which are each programmed or otherwise targeted at specific different wavelength ranges. The processor 300 may be programmed with one or more algorithms for analyzing the wavelength ranges, including minimum and maximum wavelengths for each range, through the data port 226 connected by wire to a programming device (e.g., phone 34, tablet computer 36, laptop computer 38, etc.).

At act S105, the control system 100 stores data in memory 252 at a first wavelength range. The data may include one or more pixel intensities. The first wavelength range may be a wavelength band associated with a maximum absorbance for a compound. For example, creatinine is expected to have an absorbance peak (e.g., between 500 nm and 570 nm, and or between 510 nm and 550 nm).

At act S107, the control system 100 stores data in memory 252 at a second wavelength range. The data may include one or more pixel intensities. The second wavelength range may be a wavelength band associated with a minimum absorbance for a compound. For example, the light intensity at a wavelength band at which creatinine is expected to have an absorbance minimum (e.g., between 600 and 680 nm, and/or between 360 nm and 440 nm).

At act S109, the control system 100 compares data for the first wavelength range to data for the second wavelength range. For example, the control system 100 may calculate a ratio of a value from the first wavelength range to a value of the second wavelength range.

As one alternative, the control system 100 may compare the data to the first wavelength range or the second wavelength range to a predetermined value.

At act S111, the control system 100 identify a condition based on the comparison. The condition may be that the user has an elevated concentration of the compound. The control system 100 may also generate a message including a recommendation for the user in response to the condition identified from the comparison. The recommendation may be a specific diet modification (e.g., food). The recommendation may be medical (e.g., visit a health care profession, seek further medical testing). The recommendation may be to increase water intake.

Optionally, the control system may include an input device 355. The input device may include any of the user inputs such as buttons, touchscreen, a keyboard, a microphone for voice inputs, a camera for gesture inputs, and/or another mechanism.

Optionally, the control system may include a drive unit 340 for receiving and reading non-transitory computer media 341 having instructions 342. Additional, different, or fewer components may be included. The processor 300 is configured to perform instructions 342 stored in memory 352 for executing the algorithms described herein. A display 350 may be an indicator or other screen output device. The display 350 may be combined with the user input device 355.

Processor 300 may be a general purpose or specific purpose processor, an application specific integrated circuit (ASIC), one or more programmable logic controllers (PLCs), one or more field programmable gate arrays (FPGAs), a group of processing components, or other suitable processing components. Processor 300 is configured to execute computer code or instructions stored in memory 352 or received from other computer readable media (e.g., embedded flash memory, local hard disk storage, local ROM, network storage, a remote server, etc.). The processor 300 may be a single device or combinations of devices, such as associated with a network, distributed processing, or cloud computing.

Memory 352 may include one or more devices (e.g., memory units, memory devices, storage devices, etc.) for storing data and/or computer code for completing and/or facilitating the various processes described in the present disclosure. Memory 352 may include random access memory (RAM), read-only memory (ROM), hard drive storage, temporary storage, non-volatile memory, flash memory, optical memory, or any other suitable memory for storing software objects and/or computer instructions. Memory 352 may include database components, object code components, script components, or any other type of information structure for supporting the various activities and information structures described in the present disclosure. Memory 352 may be communicably connected to processor 300 via a processing circuit and may include computer code for executing (e.g., by processor 300) one or more processes described herein. For example, the memory 352 may include graphics, web pages, HTML files, XML files, script code, shower configuration files, or other resources for use in generating graphical user interfaces for display and/or for use in interpreting user interface inputs to make command, control, or communication decisions.

In addition to ingress ports and egress ports, the communication interface 353 may include any operable connection (e.g., data port 226). An operable connection may be one in which signals, physical communications, and/or logical communications may be sent and/or received. An operable connection may include a physical interface, an electrical interface, and/or a data interface. The communication interface 353 may be connected to a network. The network may include wired networks (e.g., Ethernet), universal serial bus (USB), wireless networks, or combinations thereof. The wireless network may be a cellular telephone network, an 802.11, 802.16, 802.20, or WiMax network, a Bluetooth pairing of devices, or a Bluetooth mesh network. Further, the network may be a public network, such as the Internet, a private network, such as an intranet, or combinations thereof, and may utilize a variety of networking protocols now available or later developed including, but not limited to TCP/IP based networking protocols.

While the computer-readable medium (e.g., memory 352) is shown to be a single medium, the term "computer-readable medium" includes a single medium or multiple media, such as a centralized or distributed database, and/or associated caches and servers that store one or more sets of instructions. The term "computer-readable medium" shall also include any medium that is capable of storing, encoding or carrying a set of instructions for execution by a processor or that cause a computer system to perform any one or more of the methods or operations disclosed herein.

In a particular non-limiting, exemplary embodiment, the computer-readable medium can include a solid-state memory such as a memory card or other package that houses one or more non-volatile read-only memories. Further, the computer-readable medium can be a random access memory or other volatile re-writable memory. Additionally, the computer-readable medium can include a magneto-optical or optical medium, such as a disk or tapes or other storage device to capture carrier wave signals such as a signal communicated over a transmission medium. A digital file attachment to an e-mail or other self-contained information archive or set of archives may be considered a distribution medium that is a tangible storage medium. Accordingly, the disclosure is considered to include any one or more of a computer-readable medium or a distribution medium and other equivalents and successor media, in which data or instructions may be stored. The computer-readable medium may be non-transitory, which includes all tangible computer-readable media.

In an alternative embodiment, dedicated hardware implementations, such as application specific integrated circuits, programmable logic arrays and other hardware devices, can be constructed to implement one or more of the methods described herein. Applications that may include the apparatus and systems of various embodiments can broadly include a variety of electronic and computer systems. One or more embodiments described herein may implement functions using two or more specific interconnected hardware modules or devices with related control and data signals that can be communicated between and through the modules, or as portions of an application-specific integrated circuit. Accordingly, the present system encompasses software, firmware, and hardware implementations.

The illustrations of the embodiments described herein are intended to provide a general understanding of the structure of the various embodiments. The illustrations are not intended to serve as a complete description of all of the elements and features of apparatus and systems that utilize the structures or methods described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

While this specification contains many specifics, these should not be construed as limitations on the scope of the invention or of what may be claimed, but rather as descriptions of features specific to particular embodiments of the invention. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

Applications of the invention described herein can take the form of a computer program product accessible from a computer-usable or computer-readable medium (e.g., a non-transitory computer-readable medium) providing program code for use by or in connection with a computer or any instruction execution system, such as a computer processor of user interface device 32, computer processor 28 disposed within housing 30, or a remote cloud-based computer processor. For the purpose of this description, a computer-usable or computer readable medium can be any apparatus that can comprise, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. For some applications, the computer-usable or computer readable medium is a non-transitory computer-usable or computer readable medium.

Examples of a computer-readable medium include a semiconductor or solid-state memory, magnetic tape, a removable computer diskette, a random-access memory (RAM), a read-only memory (ROM), a rigid magnetic disk and an optical disk. Current examples of optical disks include compact disk-read only memory (CD-ROM), compact disk-read/write (CD-R/W) and DVD. For some applications, cloud storage is used.

A data processing system suitable for storing and/or executing program code will include at least one processor (e.g., a computer processor of user interface device 32, computer processor 28 disposed within housing 30, or a remote cloud-based computer processor) coupled directly or indirectly to memory elements (e.g., a memory of user interface device 32) through a system bus. The memory elements can include local memory employed during actual execution of the program code, bulk storage, and cache memories which provide temporary storage of at least some program code in order to reduce the number of times code must be retrieved from bulk storage during execution. The system can read the inventive instructions on the program storage devices and follow these instructions to execute the methodology of the embodiments of the invention.

Network adapters may be coupled to the processor to enable the processor to become coupled to other processors or remote printers or storage devices through intervening private or public networks. Modems, cable modem and Ethernet cards are just a few of the currently available types of network adapters.

Computer program code for carrying out operations of the present invention may be written in any combination of one or more programming languages, including an object-oriented programming language such as Java, Smalltalk, C++ or the like and conventional procedural programming languages, such as the C programming language or similar programming languages.

It will be understood that the algorithms described herein can be implemented by computer program instructions. These computer program instructions may be provided to a processor of a general purpose computer, special purpose computer, or other programmable data processing apparatus to produce a machine, such that the instructions, which execute via the processor of the computer (e.g., a computer processor of user interface device 32, computer processor 28 disposed within housing 30, or a remote cloud-based computer processor) or other programmable data processing apparatus, create means for implementing the functions/acts specified in the algorithms described in the present application. These computer program instructions may also be stored in a computer-readable medium (e.g., a non-transitory computer-readable medium) that can direct a computer or other programmable data processing apparatus to function in a particular manner, such that the instructions stored in the computer-readable medium produce an article of manufacture including instruction means which implement the function/act specified in the algorithms. The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide processes for implementing the functions/acts specified in the algorithms described in the present application.

The computer processors described herein are typically hardware devices programmed with computer program instructions to produce a special purpose computer. For example, when programmed to perform the algorithms described herein, the computer processor typically acts as a special purpose bodily-emission-analysis computer processor. Typically, the operations described herein that are performed by computer processors transform the physical state of a memory, which is a real physical article, to have a different magnetic polarity, electrical charge, or the like depending on the technology of the memory that is used.

It will be appreciated by persons skilled in the art that the present invention is not limited to what has been particularly shown and described hereinabove. Rather, the scope of the present invention includes both combinations and subcombinations of the various features described hereinabove, as well as variations and modifications thereof that are not in the prior art, which would occur to persons skilled in the art upon reading the foregoing description.

It is intended that the foregoing detailed description be regarded as illustrative rather than limiting and that it is understood that the following claims including all equivalents are intended to define the scope of the invention. The claims should not be read as limited to the described order or elements unless stated to that effect. Therefore, all embodiments that come within the scope and spirit of the following claims and equivalents thereto are claimed as the invention.

## Claims

1. An apparatus comprising:
one or more sensors coupled to a toilet bowl and configured to detect one or more urine-related parameters relating to urine, and
at least one computer processor configured to:
receive the one or more urine-related parameters from the one or more sensors,
determine whether the urine has a predetermined creatinine concentration based on the one or more urine-related parameters, and
determine a cause of the predetermined creatinine concentration based on the one or more urine-related parameters.

2. The apparatus according to claim 1, wherein the one or more sensors are configured to detect the one or more urine-related parameters relating to the urine, without requiring any action to be performed by any person subsequent to emission of the urine into the toilet bowl.

3. The apparatus according to claim 1 or claim 2, wherein the one or more sensors comprise one or more light sensors that are configured to detect visible light, and wherein the at least one computer processor is configured to derive a concentration of creatinine in the urine by detecting an absorbance peak that is centered in a predetermined wavelength range, within the detected visible light.

4. The apparatus according to claim 1, claim 2 or claim 3, wherein the apparatus is configured for use with an output device, and wherein, in response to determining the cause of the predetermined creatinine concentration, the computer processor is configured to generate an output on the output device indicating a recommendation.

5. The apparatus according to any one of the preceding claims, wherein the computer processor is configured to detect that the urine has an elevated concentration of urinary NT-titin, and is configured to determine that the cause of the predetermined creatinine concentration is catabolic processes at least partially in response to detecting that the urine has the elevated concentration of urinary NT-titin, optionally wherein the apparatus is configured for use with an output device, and wherein, in response to determining that the cause of the predetermined creatinine concentration is catabolic processes, the computer processor is configured to generate an output on the output device indicating an exercise recommendation.

6. The apparatus according to any one of the preceding claims, wherein the computer processor is configured to detect a specific gravity of the urine, and is configured to determine the cause of the predetermined creatinine concentration at least partially in response thereto.

7. The apparatus according to claim 6, wherein, at least partially in response to determining that the specific gravity of the urine is low, the computer processor is configured to determine that the cause of the predetermined creatinine concentration is kidney dysfunction, optionally wherein the apparatus is configured for use with an output device, and wherein, in response to determining that the cause of the predetermined creatinine concentration is kidney dysfunction, the computer processor is configured to generate an output on the output device a recommendation to seek medical attention.

8. The apparatus according to claim 6 or claim 7, wherein the computer processor is further configured to detect a concentration of urinary NT-titin of urine, and at least partially in response to determining that the concentration of urinary NT-titin and the specific gravity of the urine are both within a normal range, the computer processor is configured to determine that the cause of the predetermined creatinine concentration is dietary.

9. The apparatus according to claim 6, claim 7 or claim 8, wherein, at least partially in response to determining that the specific gravity of the urine is elevated, the computer processor is configured to determine that the cause of the predetermined creatinine concentration is either kidney dysfunction or dehydration.

10. The apparatus according to claim 9, wherein the computer processor is configured to determine a level of protein in the urine, and is configured to determine the cause of the predetermined creatinine concentration at least partially in response thereto.

11. The apparatus according to claim 10, wherein at least one of the following applies:
(i) the computer processor is configured to derive a parameter that is indicative of foaming and/or turbidity of the urine, and is configured to determine a level of protein in the urine based on the derived parameter;
(ii) at least partially in response to determining that the specific gravity of the urine is elevated and that the level of protein in the urine is elevated, the computer processor is configured to determine that the cause of the predetermined creatinine concentration is kidney dysfunction; and/or
(iii) at least partially in response to determining that the specific gravity of the urine is elevated and that the level of protein in the urine is normal, the computer processor is configured to determine that the cause of the predetermined creatinine concentration is dehydration.

12. The apparatus according to any one of the preceding claims, wherein the computer processor is configured to determine a level of protein in the urine, and is configured to determine the cause of the predetermined creatinine concentration at least partially in response thereto, optionally wherein the computer processor is configured to derive a parameter that is indicative of foaming and/or turbidity of the urine, and is configured to determine a level of protein in the urine based on the derived parameter.

13. A method comprising:
detecting one or more urine-related parameters relating to a urine sample;
determining whether the urine sample has an elevated creatinine concentration; and
determining a cause of the elevated creatinine concentration.

14. The method of claim 13, further comprising:
performing a comparison of a first intensity value for a first light spectrum to a second intensity value for a second light spectrum; and
calculating the elevated creatinine concentration based on the comparison.

15. An apparatus comprising:
one or more light sensors that are configured to receive light from a toilet bowl, while a subject's urine is disposed within the toilet bowl; and
a computer processor configured to:
determine light intensities of at least two spectral bands within the received light that are within a predetermined range, by analyzing the received light;
determine a ratio of the intensities of the at least two spectral bands within the received light; and
in response thereto, determine that the subject has elevated creatinine within their urine.
